# EUROPEAN PATENT SPECIFICATION

(11) **EP 1 861 706 B1**
(45) Date of publication and mention of the grant of the patent: **02.03.2016**
(21) Application number: 06721990.7
(22) Date of filing: 02.03.2006
(51) Int. Cl.: G01N 33/558

(54) **DEVICES AND METHODS FOR ANALYTE ASSAYS WITH BUILT-IN RESULT REPORTING USING RECOGNIZABLE SYMBOLS**
VORRICHTUNGEN UND VERFAHREN FÜR ANALYTTESTS MIT EINGEBAUTER ERGEBNISMITTEILUNG UNTER VERWENDUNG ERKENNBARER SYMBOLE
DISPOSITIFS ET PROCÉDÉS D'ANALYSE D'ESSAIS À RAPPORTS DE RÉSULTATS INTÉGRÉS UTILISANT DES SYMBOLES RECONNAISSABLES

(30) Priority: 03.03.2005 CN 200510049177; 03.03.2005 US 73028
(43) Date of publication of application: 05.12.2007
(73) Proprietor: Alere Switzerland GmbH, 6300 Zug (CH)
(72) Inventor: TANG, Zuifen, Hangzhou, Zhejiang 310023 (CN); XIONG, Dengfeng, Hangzhou, Zhejiang 310023 (CN); WU, Shujiang, Hangzhou, Zhejiang 310023 (CN); GUAN, Zhumin, Hangzhou, Zhejiang 310023 (CN)
(74) Representative: Lee, Nicholas John
(86) International application number: PCT/CN2006/000310
(87) International publication number: WO 2006/092103

(56) References cited:
- EP-A- 0 362 809
- EP-A- 0 505 636
- WO-A-03/058245
- WO-A-2004/003559
- WO-A1-94/01775
- WO-A2-01/81457
- CN-A- 1 340 707
- CN-A- 1 465 977
- US-A- 5 130 290
- US-B1- 6 194 224

## Description

### Field of the Invention

The present invention is directed to devices for the detection of an analyte and, in certain embodiments, to the presentation of test results as recognizable symbols.

### Background of the Invention

The following Background of the Invention is intended to aid the reader in understanding the invention and is not admitted to be prior art.

The inclusion of positive and negative control tests in the performance of an assay is considered an important component of any assay. In immunological tests, these control tests are often performed by including an analyte binding control test in the assay, which may appear as a colored line on the test strip. These types of control tests are effective for verifying that the assay device is functioning correctly, but they also result in added expense in making the device and performing the assay, particularly when the specific binding molecules used in the control test are produced as a result of elaborate procedures. Additionally, these types of controls can be confusing for the untrained general consumer and lead to improper test interpretation. There is therefore a need for better methods and apparatuses for performing sample collection and testing.

### Summary of the Invention

In a first aspect, the present invention provides a device for detecting the presence or absence of an analyte in a sample comprising:
a matrix that supports the flow of a liquid sample;
a sample application zone on the matrix for receiving a liquid sample;
a detection zone on the matrix having
   a positive control area comprising a positive control filament containing one or more components that exhibit a first color when dry and a second color when wet, the one or more components comprising a colourless or pale-coloured basic dye selected from the group consisting of a triarylmethane-based dye, a diphenylmethane-based dye, a thazine-based dye, a lactam-based dye, and a fluoran-based dye, wherein the positive control filament is an independent structure on the device; and
   an analyte binding area comprising a specific binding molecule; and one or more reagent zones on the matrix comprising reagents for conducting the assay.

In a second aspect, the present invention provides a method of determining the presence or absence of an analyte in a liquid sample comprising; placing the liquid sample onto the device of the first aspect;
allowing the liquid sample to flow through the matrix and thereby pass through the one or more reagent zones so that reagents for conducting the assay react with the liquid sample to form a detectable reaction product when analyte is present in the liquid sample;
allowing the liquid sample to flow through the detection zone thereby wetting the positive control filament comprising one or more components that exhibit a first color when dry and a second color when wet, wherein analyte contained in the sample is restrained on the analyte binding area as sample flows through the detection zone; and
observing the detection zone of the device to determine the presence or absence of analyte in the liquid sample.

The present invention provides devices and methods for detecting the presence of an analyte in a liguid sample and indicating to the user the present or absence of the analyte for example using recognizable symbols. In one embodiment, within the detection zone are located positive and negative control areas, and an analyte binding area. The positive control area contains one or more components that exhibit a first color when dry and a second color when wetted. The analyte binding area contains binding molecules that capture an analyte, which can be a labeled analyte. The analyte binding area and positive control area interact with each other to form a recognizable symbol and thereby provide the test result. In one embodiment, the positive control area is present on the matrix in the shape of a minus sign, and the one or more components are selected so that the positive control area will change from white to red upon exposure to an aqueous solution. In this embodiment, the assay begins with a dry test strip and the positive control area and the test strip are both white in color. Therefore, before sample is applied, the positive control area is not apparent upon observation of the detection zone. When liquid sample is applied, it flows through the matrix and through the detection zone, thereby wetting the positive control area as it passes through, and thus causing the positive control area to turn from white to red. Therefore, a minus sign becomes visible in the detection zone. If no analyte is present in the sample, only the minus sign is visible in the detection zone and a negative test result is communicated to the user. However, if analyte is present in the sample, the labeled analyte is captured in the analyte binding area and the labeled analyte accumulates and provides a color, and the analyte binding area interacts with the minus sign (the positive control area) to form a plus sign, thus communicating a positive test result to the user. The symbols selected can be any recognizable symbols, for example, a plus sign, a minus sign, an "X" or another symbol known in the art or in general parlance as conveying a particular meaning.

The matrix can be a nitrocellulose assay strip, and the positive control area can be comprised in the shape of a minus sign situated on the assay strip. The analyte binding area can comprise two areas situated on either side of the positive control area and having a specific binding molecule that binds to the analyte or to a molecule bound to the analyte. The two areas are aligned so that the positive control area and analyte binding area interact to form a recognizable symbol when the positive control area is wet and analyte is present in the sample.

The recognizable symbol can be a plus sign or any recognizable symbol. Many examples of colorless or pale-colored basic dyes are described in U.S. Patent No. 5,130,290.

The specific binding molecule can be an antibody or antibody fragment. In one embodiment, the analyte is human chorionic gonadotropin (hCG) or luteinizing hormone. The positive control area can be demarcated by one or more positive control filaments present in the detection zone, and the positive control area may not contain a member of a specific binding pair.

In another embodiment, the analyte binding area further contains a specific binding molecule that specifically binds to the analyte and that may have a label that provides a detectable signal. The label can be a colored particle, such as a dextran bead. The analyte binding area can comprise a bar situated latitudinally along the axis of the strip, and further comprise a specific binding molecule for the analyte or for a molecule bound to the analyte. The positive control area can comprise two areas situated at either side of the analyte binding area, and the positive control area and analyte binding area can interact to form a recognizable symbol.

In another embodiment the matrix is a test strip comprised of a bibulous material, the positive control area is comprised in the shape of a minus sign situated longitudinally along the axis of the bibulous material, and the analyte binding area comprises two areas situated on either side of the positive control area, and the analyte binding area and positive control area interact to form a recognizable symbol. The analyte binding area may comprise the shape of a bar situated latitudinally along the axis of the strip, and the analyte binding area may comprise a specific binding molecule for the analyte bound to a label.

In the method of the invention, analyte present in the sample may be labelled with a detectable label as it passes through the reagent zone, and labelled analyte may be retained at the analyte binding area as sample passes through the detection zone.

Disclosed herein are kits for determining the presence or absence of an analyte in a fluid. The kits contain a device of the present invention, as described herein, and instructions for use of the device. The test device of the kit can be configured to test for the presence of hCG in a sample, and the instructions explain how to use the device to test for the presence of hCG in urine. In other embodiment, the device can be configured to test for the presence of luteinizing hormone (LH) or follicle stimulating hormone (FSH) in a biological fluid, and the instruction provided in the kit describe how to use the device to test for the presence of these substances.

The summary of the invention described above is not limiting and other features and advantages of the invention will be apparent from the following detailed description, as well as from the claims.

### Brief Description of the Drawings

Figure 1A provides a perspective view of a device having a matrix of **20.** The matrix includes a sample application zone **25,** a reagent zone **30,** and a detection zone **32.** The detection zone contains a positive control area **45** present in the shape of a minus sign. The positive control area **45** has been printed or painted on the nitrocellulose **35.** The detection zone also contains an analyte detection area **40** and a negative control area **12.** An arrow indicates the direction of sample flow.
Figure 1B provides a perspective view of another device. In this device, the matrix **20** is composed of a multiple materials, which are in fluid communication with each other. The positive control area **45** is present in the shape of a minus sign, and is striped, printed, coated, or painted on the nitrocellulose.
Figure 2A is an exploded view of an embodiment of a device of the present invention, in which the positive control area comprises a filament **50.** The one or more components that provide a first color when dry and a second color when wet are contained on or in the filament. In this embodiment, the filament fits within a groove **55** cut in the nitrocellulose.
Figure 2B illustrates an embodiment related to that shown in Figure 2A. In this embodiment, the positive control area comprises a filament **50** situated on top of the nitrocellulose. The one or more components are contained on or in the filament.
Figure 2C illustrates an embodiment similar to that shown in Figure 2B, except that the filament **50** is present underneath the nitrocellulose. When wet, the filament (exhibiting the second color) is visible in the detection zone.
Figure 3A shows an exploded view of another embodiment of a device of the invention. In this embodiment, the positive control area is demarcated by a filament **50** present below the nitrocellulose. In this embodiment, the filament takes the form of a tongue protruding from a positive control pad **52** and fits into the groove **85** of a support layer **80.**
Figure 3B illustrates the positive control portion of the embodiment shown in Figure 3A. The filament **50** is a tongue that protrudes from the positive control pad **52.** The filament (tinted area) has been treated with one or more components that change from a first color to a second color when wetted. The filament fits into a groove **85** cut into the support layer **80.**
Figure 4A provides an exploded view of yet another embodiment of the present device. In this embodiment, the filament **50** is present in the matrix below the nitrocellulose **35,** and the test strip includes a bottom support **60.** The filament **50** is in liquid communication with the reagent pad **30.** In certain embodiments, a cover pad **2** may be used to promote contact between the sample pad, reagent pad and nitrocellulose underlying the cover pad.
Figure 4B illustrates an embodiment of the invention with a groove **51** cut into the bottom support **60.** The filament **50** is placed into the groove and nitrocellulose **35** is situated on top of the groove.
Figure 4C illustrates the appearance of the test results before use (Fig. 4C *i*), when the test results are negative (Fig. 4Cii) and when the test results are positive (Fig. 4C *iii*)*.*
Figure 5A is an exploded view of yet another embodiment of the present device. In this embodiment, the filament **50** projects from a positive control pad **52** and is present under the nitrocellulose **35.** In this embodiment a clear support layer **54** is present between the positive control pad **52** and filament **50,** and the nitrocellulose **35.**
Figure 5B provides an assembled perspective view of the device shown in Figure 5A.
Figure 6A is an exploded view of a further embodiment of the present device, having two positive control pads **52** and two filaments **50.** Additionally, there is a support **80** with a groove **85** sized and shaped to support the two filaments **50.**
Figure 6B provides an assembled perspective view of the device shown in Figure 6A. An optional sample collection pad is shown as dashed lines.

### Detailed Description

In the following detailed description, reference is made to the accompanying drawings that form a part hereof, and in which is shown by way of illustration specific embodiments in which the invention may be practiced. It is understood that other embodiments may be utilized and structural changes may be made without departing from the scope of the present invention.

### Test Devices

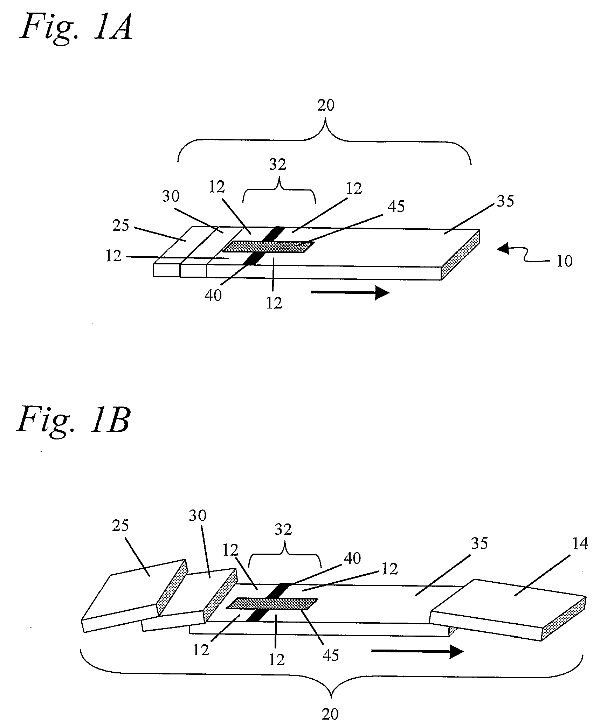

In one embodiment the devices of the invention utilize test strips to detect the presence of an analyte in a liquid sample. The devices convey the test results to the user with recognizable symbols that are formed by the interaction of visible signals from the positive control area and the analyte binding area. Figure 1A illustrates a device 10, comprising a test strip having a matrix 20 that supports the flow of a liquid sample. The matrix includes a sample application zone **25** where liquid sample is applied to the device, a reagent zone **30** and a detection zone **32.** The reagent zone **30** contains reagents for conducting the assay, and more than one reagent zone can be present on the test strip, depending on the requirements of the particular assay being conducted. The detection zone **32** includes a positive control area **45,** an analyte binding area **40,** and a negative control area **12.** The negative control zone is an area where color should not form if the assay is functioning correctly. In this device the negative control zone **12** is conveniently designated by those areas surrounding the plus sign that forms when analyte is present in the sample (i.e., when a plus sign is selected as the recognizable symbols).

Figure 1B illustrates another device, in which the matrix **20** is composed of multiple overlapping materials, including a sample application pad **25,** a reagent pad **30,** a nitrocellulose strip **35** and an absorbent pad **14.** The detection zone **32** is located on the nitrocellulose strip. The positive control area and negative control area are located within the detection zone. The absorbent pad provides an absorbent sink to absorb liquid sample and therefore promote liquid flow through the matrix until the conclusion of the assay, In The matrix components can be supported by one or more adhesive backings (not shown).

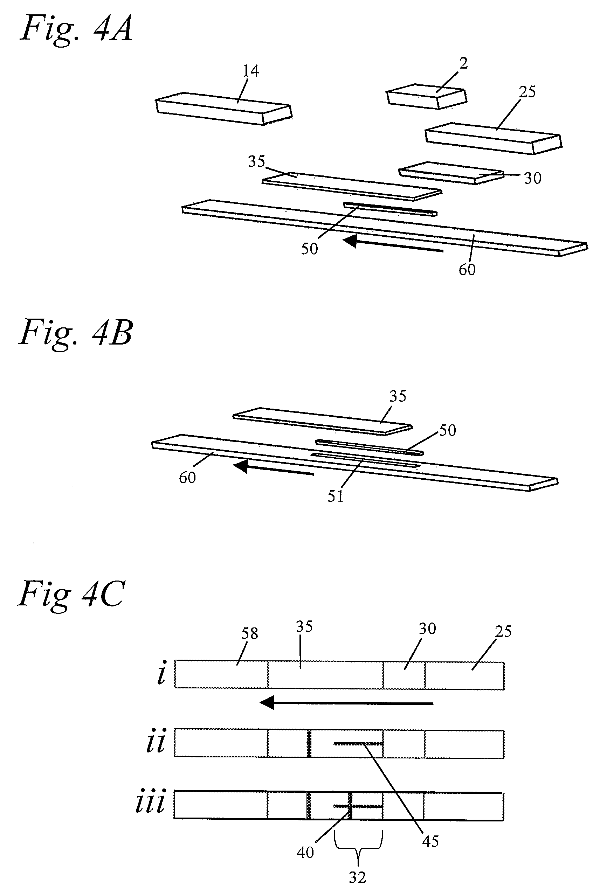

In one device of the invention (Figure 4a), the positive control filament **50** is treated with a water-sensitive ink and positioned between a nitrocellulose strip **35** and a backing **60.** In this embodiment the filament **50** is made of a bibulous paper, which has been impregnated with a water-sensitive reagent. The filament **50** is placed across the axis of the analyte binding area so that, in the event of a positive result, the positive control area interacts with the analyte binding area to form a plus sign. In this embodiment, one side of the filament **50** is underneath the nitrocellulose strip **35** and the other side of the filament is in liquid communication with the reagent pad **30** or the sample application pad **25.** A "positive control filament" refers to a structure ofthe device that contains the one or more components that provide a first color when dry, and a second color when wetted. The filament need not take any particular shape, but will usually be in the shape of an elongated member on the device visible in the detection zone. The shape of the filament will depend on the recognizable symbol(s) use in the assay. The filament can be present as an independent structure on the device.

In another embodiment (Figure 4b) the filament is placed into a groove **51** cut into the backing **60,** and also lies across the axis of the analyte binding areas, so that a plus sign forms when analyte is present in the sample.

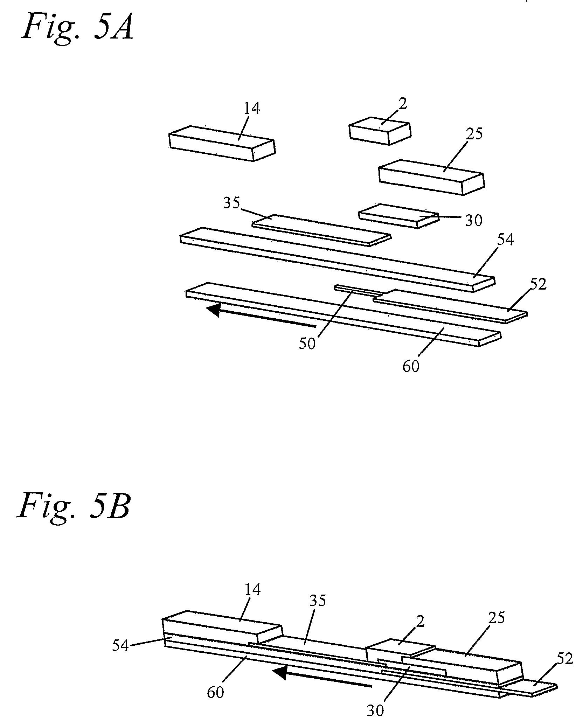

In another embodiment (Figure 5) the positive control filament **50** is present as an extension of a positive control pad **52.** The filament **50** extends underneath the nitrocellulose and crosses the axis of the analyte binding areas **40,** to form the plus sign when analyte is present in the sample and the minus sign when no analyte is present in the sample. In this embodiment the filament **50** is impregnated with the color-changing reagent, and the filament **50** and positive control pad **52** are in fluid communication with each other and with the reagent zone **30** and sample application zone **25.** Therefore, when fluid sample is applied to the sample application zone **25,** the positive control pad **52** and filament **50** become wet, thereby causing the color-changing reaction to occur. The filament **50** interacts with the analyte binding area to form the recognizable symbol.

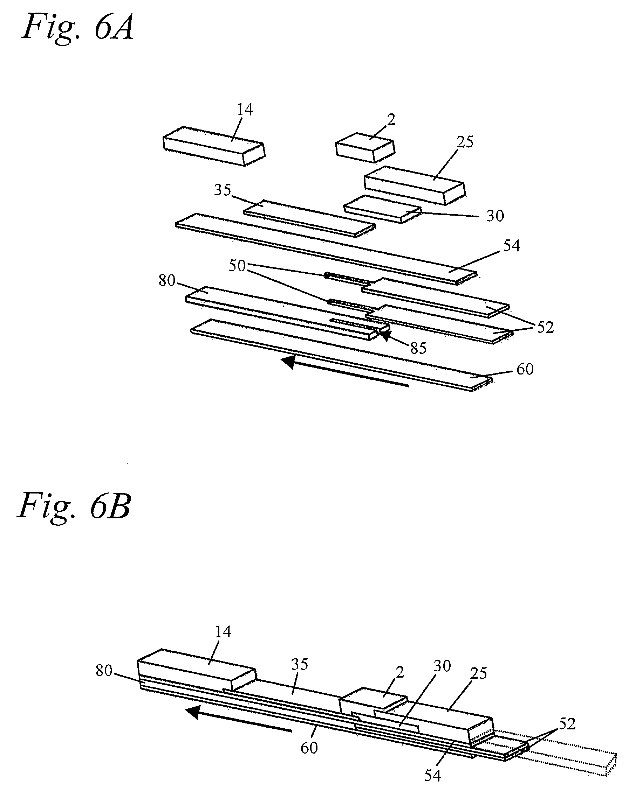

In the embodiment depicted in Figure 6, the device contains an additional support layer **80** to support the filament **50.** The filament **50** is inserted into a groove **85** cut in the support layer **80.** The backing **60** can be attached (e.g., by adhesive) to the support layer **80.**

The devices of the invention can be provided in the form of a test device, featuring a test strip and a holder for holding the test strip. The holder can be a hollow plastic body with windows located to allow viewing of the detection zone and access to the sample application zone. In another format the devices are provided as a test strip, and no holder is included. The holder can also be located only at one end of the test strip to enable a user to pick it up without contamination of the device, and the sample application zone placed into a sample solution.

In some formats the devices can also include a control line, in addition to the positive control area. In these formats the control line can appear upstream or downstream of the positive control area. A positive result is determined not only by viewing the detection zone for the presence of a recognizable symbol, but also by comparing the analyte binding area to the control line. In some assays the relative intensities of the two lines are compared to determine a positive or negative result for the assay. In one format of this embodiment the analyte is luteinizing hormone.

### Matrix

In one embodiment the test strip contains a bibulous material providing a matrix to support the flow of liquid. "Matrix" refers to a material that supports the flow and transport of fluid. In one embodiment the matrix is a bibulous material. The flow of fluid through the device can be by force of capillary action. In different embodiments the matrix can be a strip of a single material (Figure 1A) or the matrix maybe assembled from multiple bibulous materials that are in fluid communication with each other (Figures 1B-6). "Bibulous" materials are those that readily absorb liquid and through which liquid is transported by capillary action. Examples of bibulous materials include nitrocellulose, filter paper, glass fibers, polyester, and other suitable materials.

### Sample Application Zone

The sample application zone can contain a buffer for solubilizing the sample, or can be simply a location on the matrix for the application of sample, but it also can contain other reagents for conducting the assay. For example, "scavenger" antibodies can be present in the sample application zone, the reagent zone, or other zones of the matrix in those embodiments where they are useful. The sample application zone can therefore also be a reagent zone. Sample is advantageously applied in a liquid form to begin the assay, but can also be dried on the test strip and the assay begun by applying water, buffer, or other reagents to solublize the sample and begin the assay. The sample itself can be a liquid sample or a solid sample that has been liquefied or otherwise prepared in a liquid form.

### Reagents

The reagents contained in the reagent zone can be movably present in the reagent zone. Some reagents can be attached to a label and bind to analytes of interest present in the sample, thereby providing a labeled analyte. The sample application zone and/or reagent zone can also contain buffers for solubilizing the sample or adjusting the pH, as may be required in the specific assay. In one embodiment the reagent zone contains a specific binding molecule (e.g., an antibody or antibody fragment) linked to a label. The label can be any convenient label, such as a gold sol, a fluorescent dye, or a water soluble dye. The specific binding molecule can bind specifically to one or more epitopes on the analyte of interest, thereby labeling the analyte.

### Detection Zone

The detection zone of the device contains the positive control area, negative control area (when present), and the analyte binding area. The negative control area is that space located in the detection zone that is not a part of either the positive control area or analyte binding area. If a detectable signal from the detectable label is detected in this area, the assay is invalid due to a failed negative control. In some embodiments the detection zone is a rectangle or square on a bibulous matrix that encompasses the length of the positive control area or analyte binding areas, measuring longitudinally along a test strip, and is further encompassed by lines drawn perpendicular to the sides of the test strip. The detection zone can also have a plastic part of the device placed over it to provide a viewing window limited within the detection zone. The "detection zone" refers to a view of the device. Thus, structures can be contained in the detection zone whether they are physically located on, in, or under the matrix, as long as they are visible upon observation of the detection zone in either a wet or dry state, or as the result of a positive or negative analyte assay result,

### Positive Control Area

The positive control area can be delineated by one or more areas on the device that contain the one or more color-changing components that exhibit a first color when dry and a second color when wet. The positive control area can be present as a bar situated longitudinally along the axis of the strip. The bar can be delineated by a material containing the color-changing components. In another embodiment the color-changing components can be affixed to the matrix or backing or other support, to delineate the positive control area. For example, the color-changing components can be attached to a substance that is affixed to the matrix or backing or other support, and can also be affixed directly to these structures. In one embodiment the color-changing components can be attached to a protein, which is itself attached to the nitrocellulose portion of the matrix.

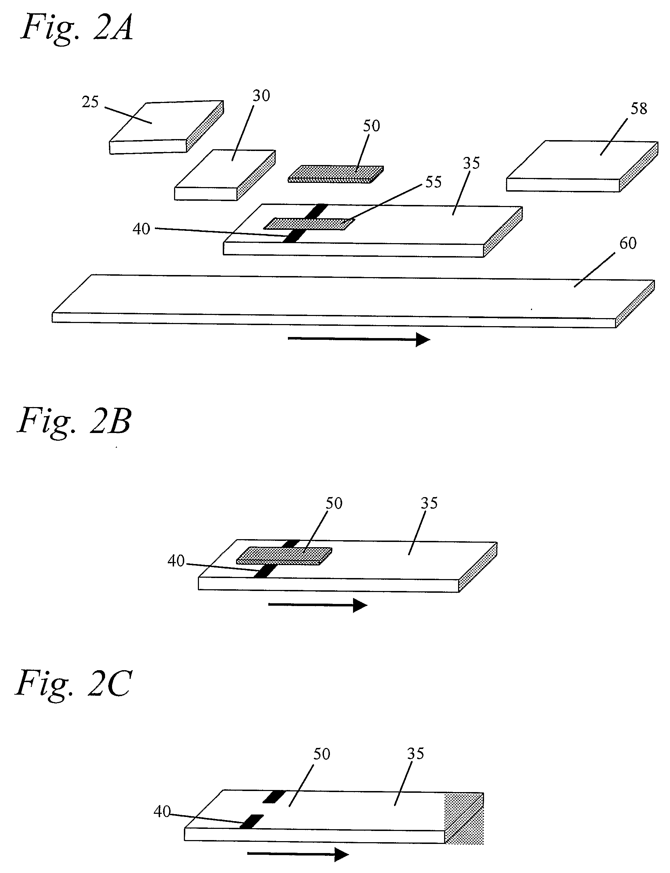

In certain embodiments, the positive control area is delineated by a positive control filament. The filament can be made of any suitable material that canretain the color changing components, so that they can exhibit a second color when wet. In some examples the filament is made of paper or another fibrous or cellulosic material that can carry fluid. The filament can be situated to be a part of the matrix, since it will carry fluid. Figure 2A shows a matrix composed of multiple bibulous materials. The matrix is supported by a backing **60,** which in one embodiment contains an adhesive. The adhesive helps to hold the parts of the matrix together and thereby retain fluid communication between the parts. A variety of adhesives can be conveniently used, such as medical grade glue or adhesive strips and double-sided tape. In the embodiment illustrated in Figure 2A, a nitrocellulose strip **35** is located on top of the backing **60.** In different embodiments the nitrocellulose strip **35** can be supported by its own backing (e.g., MYLAR^{®}) or can be placed on the backing **60** containing adhesive, without a separate backing. In this embodiment, the matrix also has a reagent pad **30,** a sample application pad **25** and an absorbent pad **58.** The components of the matrix abut and slightly overlap each other so that applied sample flows continuously from the application pad to the absorbent pad. The detection zone contains the analyte binding area **40** and a positive control filament **50.** The filament contains the one or more components that exhibit a first color when dry and a second color when wetted. In this embodiment a groove **55** sized and shaped to fit the filament, is cut in the nitrocellulose. This groove **55** may be cut in the nitrocellulose. Alternatively, a groove can be cut through the backing of the nitrocellulose, if present. During construction of the test strip, the filament is placed in the groove in the nitrocellulose. The reagents can be applied to the analyte binding area of the nitrocellulose either before or after cutting the groove or placing the filament in the groove.

Figures 2B and 2C show related embodiments. In the embodiment illustrated in Figure 2B, the filament is placed on top of the nitrocellulose, instead of in a groove cut into the nitrocellulose. In the embodiment illustrated in Figure 2C, the filament is placed below the nitrocellulose layer. The filament can be located between the nitrocellulose and the backing of the nitrocellulose (if present), or can also be located between the nitrocellulose and an adhesive backing (when the nitrocellulose does not have its own backing). When wet, the nitrocellulose is translucent or transparent, such that the wetted colored filament **50** is easily visible through the nitrocellulose.

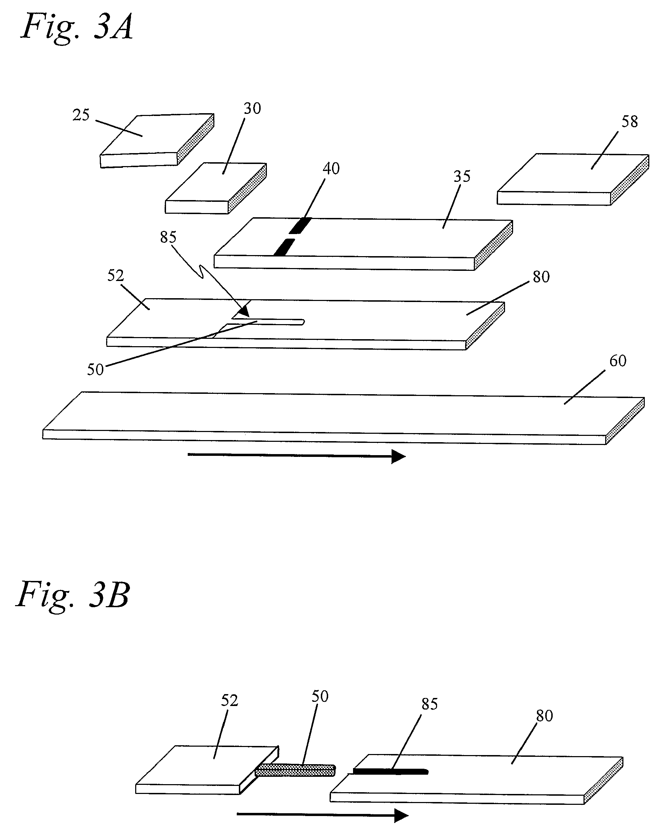

Figures 3-6 illustrate more embodiments of the device, in which the filament is present in the shape of a positive control filament **50** protruding from a positive control pad **52.** Referring to Figure 3A, the filament **50** fits into a groove **85** in a support layer **80** (e.g., a plastic strip). The positive control filament **50** can contain a dye containing one or more components that exhibit a first color when dry, and a second color when wetted. The nitrocellulose can be positioned on a support layer **80** and positive control pad **52,** such that when the positive control pad **52** becomes wet with sample, the fluid sample flows to the positive control filament **50,** causing it to change from the first color to the second color. In one embodiment, when a color-changing dye containing the one or more components is used, it can be formulated to change from white (first color) to red (second color). When the filament turns to the second color, it becomes visible through the overlaying nitrocellulose, which has also been wet by the sample. A backing on the nitrocellulose (e.g., MYLAR^{®}) and/or a transparent support **54** (Figures 5 & 6) can be used to prevent sample migration out of the filament.

Various bibulous materials can be used as the positive control filament and/or positive control pad (when utilized), which can be constructed of the same material. Any material that has the ability to transport liquid can be used. The liquid can move through the material by force of capillary action. In one embodiment the positive control pad or positive control filament is a polyamide fiber. Membrane thicknesses of between 0.6 and 1.0 mm are useful, but other thicknesses can be used as convenient. The material is absorptive in its nature, and in this embodiment a polyamide fiber material of 60 mm x 10 mm will absorb 0.6 grams of fluid, +/- 0.15 grams. Useful polyamide fibers are available as wicking material from Filtrona Fibertec™ (Colonial Heights, VA). Of course other bibulous materials can also be useful in the invention. For example, surface active media that are often used as filtering materials and utilize either amine or carboxyl groups on the surface of the fiber as substrates for a wide variety of linking agents can also be used. This material also functions well in the present invention as bibulous material when supplied as a sheet or strip, and is also available from Filtrona Fibertec™ (Colonial Heights, VA). Other embodiments include, but are not limited to, the use of cotton fiber. Polyester is another material that is useful as a bibulous material and is advantageously treated according to methods known in the art with detergents, proteins, and buffers.

### Symbols

Recognizable symbols may be created by the interaction of the positive control and analyte binding areas on the device. The positive control area can be delineated by choosing a portion of a symbol that will interact with the analyte binding area, and affixing the shape to form the positive control area. The symbol can also be selected to present one recognizable symbol for a negative result, and another recognizable symbol for a positive result, where the positive control area and analyte binding area interact to form a recognizable symbol in the event of a positive assay result. The positive control area contains one or more components that exhibit a first color when dry and a second color when wetted. The symbol (or portion of a symbol) can be affixed to form the positive control area by printing or painting the symbol onto the matrix (and attaching the one or more components that exhibit a first color when dry and a second color when wet to the material to be affixed), or by other methods known in the art. The symbol can be formed above, below, or within the nitrocellulose, or located between the nitrocellulose and a backing for the nitrocellulose.

In various embodiments the "recognizable symbol" can be a plus sign, a minus sign, a dash, a bar, an "X," or another symbol known in the art or in general parlance as conveying a particular meaning that can be associated with the assay result. Any meaningful symbol can be selected, such as a letter from the Roman alphabet, a number, a mathematical operator, a scientific symbol, or a letter from another language or alphabet system, for example a letter from the Chinese, Japanese, or Arabic alphabets. For example, a minus sign is advantageously used to indicate a negative result, because it is a meaningful and easily recognized symbol, and can also be conveniently configured to interact with an analyte binding area to form a plus sign. Other symbols, such an "X," "O," null sign, "Y," "N," "Z," or an arrow, can also be selected. These symbols can be easily read and understood by an untrained user. When the one or more components that change color and the demarcation of the positive control area are selected to be the same color, the recognizable symbol is formed by the interaction of the positive control area and the analyte binding area when a positive result is obtained. When the symbol is a minus sign, it can have either square or rounded edges.

Figures 4C, i-iii illustrate an embodiment where the recognizable symbol is a plus sign and a minus sign, indicating a positive and negative assay result, respectively. As shown in Figure 4Ci, prior to use no symbol can be seen in the detection zone **32.** If no analyte is present in the sample a minus sign appears in the positive control zone **45,** indicating a negative result (Figure 4Cii). When analyte is present in the sample a plus sign appears through the interaction of the positive control zone and the analyte detection zone **40** (see Figure 4Ciii), indicating a positive result.

### Color-Changing Material

The one or more color-changing components comprise a colorless or pale-colored basic dye selected from triarylmethane-based dyes, diphenylmethane-based dyes, thazine-based dyes, lactam-based dyes, and fluoran-based dyes. Numerous color-changing components are described in U.S. Patent No. 5,130,290 to Tanimoto. These components can be combined to form an ink or other water sensitive coloring layer that can be applied to a substrate (e.g., a paper or portion of an assay device). In some embodiments, the color-changing components include a colorless or pale-colored basic dye, a color developing material that forms a color on contact with the dye, a desensitizer, and a binder. A wide variety of dyes, color developing materials, desensitizers, and binders may be combined in various ratios to produce an ink with the desired color changing capabilities, and many examples of suitable components and combinations of components are described in U.S. Patent No. 5,130,290. The one or more components can be formulated as a water sensitive ink. The ink or other water sensitive layer material can also be purchased from Kanzaki Paper Manufacturing Co., Ltd., Tokyo, Japan, Beijing Chuang-Xin Cheng-Ye Tech. Ltd., AD: #02-4-2, Yungang South Lane, Fengtai District, Beijing, China, or Shenzhen Huiiu Tech. Ltd., Shenzhen, China. Examples of color developing materials include 4-tert-butylphenol, alpha-naphthol, beta-naphthol, 4-acetylphenol. Examples of desensitizers include polyethyleneimines, polyoefin glycols, anionic surfactants, and nonionic surfactants. Examples of binders include starches, hydroxyethyl cellulose, methyl cellulose, ethyl cellulose, carboxymethyl cellulose, gelatincasein, gum arabic, and water-soluble polymers. Additional examples of each are found at U.S. Patent No. 5,130,290.

In one embodiment, the one or more components of the ink produce a white color when dry, and a red color when wet. In other embodiments, an orange or blue color is produced when the material is wetted, but the materials can be selected so that many colors can be formed as desired. The white or colorless ink can be striped onto, coated, printed, or otherwise applied in the detection zone of the device or to the positive control filament. The positive control filament prior to use may not be visible to the user, since it is the same color as the matrix, and can also be present within or underneath the nitrocellulose, or in another layer of the fluid-carrying matrix. When the sample flows through the positive control area, the one or more components of the ink are wetted and become red, making the positive control area visible in the shape of a minus sign. In other embodiments, the first color of the positive control area can be a color other than white, and the device changes from a first color to a second color. Any colors desired and for which components are available can be selected as the first and second colors.

### Analyte Binding Area

The analyte binding area may be positioned on the matrix so that it interacts with the positive control area to provide a recognizable symbol when the analyte of interest is present in the liquid sample. Labeled reagents present in a reagent zone can bind (directly or indirectly) to the analyte of interest, thereby labeling the analyte of interest with a detectable label as it flows through the matrix. The analyte binding area can also contain reagents that bind to a moiety associated with the analyte. That moiety can be an immunological epitope on the analyte itself, or on a reagent bound to the analyte (e.g., a reagent that bound to the analyte as it passed through the reagent zone). In various embodiments the reagent bound to the analyte can be an antibody, a fragment or portion of an antibody, an antibody (or fragment thereof) derived from a species different from the antibody affixed to the analyte binding area, or another member of a specific binding pair, for example, avidin, streptavidin, or biotin, which itself can be bound to a moiety bound to the analyte.

In one embodiment the analyte binding area can be two areas situated on either side of the positive control zone so that when analyte is present in the sample, it is labeled during the assay and is retained at the analyte binding area, and forms the recognizable symbol for a positive assay by interacting with the positive control area. In another embodiment, the analyte binding area is a bar situated latitudinally along the axis of the strip, and contains a specific binding molecule for the analyte, or for a molecule bound to the analyte. In either case, when labeled analyte is present in the sample as it moves through the detection zone, it accumulates at the analyte binding area to provide a detectable color in the analyte binding area. The interaction between the color at the analyte binding area and the positive control area provides the recognizable symbol. In some embodiments the label is a colored particle, which may be a dextran bead, gold sol, or other labeling particle, but the label can be any suitable label that provides a detectable signal.

### Reagent Zone

The label that binds the analyte of interest serves to provide the visually detectable signal in the analyte binding area, which will interact with the positive control area to form the recognizable symbol when analyte is present in the sample. Specific binding molecules for the analyte carrying a label can be present in the reagent zone. When the specific binding molecules capture the analyte, and when the labeled analyte is bound within the analyte binding area, the area becomes visible due to the accumulation of the label in the area. A "specific binding molecule" for the analyte refers to a binding molecule that binds to the analyte and does not substantially bind to any other molecule present in the sample. The specific binding molecule for the analyte can also bind to a molecule that correlates with or indicates the presence of analyte in the sample. By substantial binding is meant that binding occurs to an extent that will change or obscure the result of the assay. In some embodiments the specific binding molecule can be an antibody or an antibody fragment (e.g., the Fab region of an antibody), an antigen, a receptor or fragment of a receptor that binds a ligand, or a member of a biotin-streptavidin pair or other type of binding pair.

A label can thus be provided in the reagent zone, and as the sample flows through the reagent zone the analyte is bound with a label that provides a detectable signal. A "label pad" is an area of the matrix where there is present a label for the analyte suspected of being present in the sample. Therefore, a reagent zone can be a label pad. The "label" can be any suitable label that provides a detectable signal. For example, the label can be a sol particle, a fluorescent molecule, a chemiluminescent molecule, a metal or alloy (e.g. colloidal gold), or a sac, in particular a liposome containing a visible dye. Also useful are hydrophobic sols, which hydrophobic organic dyes or pigments are insoluble in water or soluble only to a very limited extent. The label can also be polymer particles, such as colored polystyrene particles (e.g., spherically shaped). Other useful particulate labels include ferritin, phycoerythrins or other plrycobili-proteins, precipitated or insoluble metals or alloys, fungal, algal, or bacterial pigments or derivatives such as bacterial chlorophylls, or other plant materials. In certain embodiments, the label is a colored particle, such as a dextran bead. In other embodiments, the label and the dye used for the positive control are selected to have similar colors, to enhance the interaction of the two signals in producing a single apparent symbol on or in the matrix.

In other embodiments, the label can be a labeled specific binding molecule (e.g., an antibody) for the analyte. For example, in one embodiment the analyte of interest is human chorionic gonadotropin (hCG), and the label that attaches to the hCG is gold-sol labeled anti-hCG antibody. When the sample reaches the reagent zone (or label pad), the hCG present in the sample is bound by the gold-anti-hCG antibody. The labeled antibody does not interfere with the binding of capture molecule present in the analyte binding area, which binds the labeled hCG. For example, the label can bind one portion of the analyte and the capture molecule can bind another portion of the analyte, or can bind the label. The hCG-anti-hCG antibody-gold complex migrates downstream in the matrix. When the complex reaches the analyte binding area the capture molecule binds to form a complex of gold-anti-hCG antibody-hCG-anti-hCG antibody. The capture molecule can be another specific binding molecule for hCG, or a specific binding molecule for a moiety bound to the hCG analyte. When the gold-anti-hCG specific binding molecule-hCG-anti-hCG specific binding molecule complex is bound to the analyte binding area, the analyte binding area is colored by the gold label on the complex and therein becomes visible to the unaided eye. In one embodiment the specific binding molecules are antibodies or antibody fragments. The labeling and capture binding molecules can bind to different epitopes on the analyte. In one embodiment the labeled specific binding molecule binds to beta-hCG, and the capture binding molecule binds to alpha-hCG.

"Antibody" refers to an immunoglobulin, whether natural or partially or wholly synthetically produced. The term also includes derivatives thereof which maintain specific binding ability. The term also covers any protein having a binding domain which is homologous or largely homologous to an immunoglobulin binding domain. These proteins may be derived from natural sources, or partly or wholly synthetically produced. An antibody may be monoclonal or polyclonal. The antibody may be a member of any immunoglobulin class, including any of the human classes: IgG, IgM, IgA, IgD, IgG, and IgE. An "antibody fragment" is any derivative or portion of an antibody which is less than full-length. The antibody fragment can retain at least a significant portion of the full-length antibody's specific binding ability. Examples of antibody fragments include, but are not limited to, Fab, Fab', F(ab')₂, scFv, Fv, dsFv diabody, and Fd fragments.

The antibody fragment may be produced by any means. For instance, the antibody fragment may be enzymatically or chemically produced by fragmentation of an intact antibody or it may be recombinantly produced from a gene encoding the partial antibody sequence. Alternatively, the antibody fragment may be wholly or partially synthetically produced. The antibody fragment may optionally be a single chain antibody fragment. Alternatively, the fragment may comprise multiple chains which are linked together, for instance, by disulfide linkage. The fragment may also optionally be a multimolecular complex. A functional antibody fragment will typically comprise at least about 50 amino acids and more typically will comprise at least about 200 amino acids.

Single-chain Fvs (scFvs) are recombinant antibody fragments consisting of only the variable light chain (V_{L}) and variable heavy chain (V_{H}) covalently connected to one another by a polypeptide linker. Either V_{L} or V_{H} may be the NH₂ -terminal domain. The polypeptide linker may be of variable length and composition so long as the two variable domains are bridged without serious steric interference. Typically, the linkers are comprised primarily of stretches of glycine and serine residues with some glutamic acid or lysine residues interspersed for solubility. "Diabodies" are dimeric scFvs. The components of diabodies typically have shorter peptide linkers than most scFvs and they show a preference for associating as dimers.

An "Fv" fragment consists of one V_{H} and one V_{L} domain held together by noncovalent interactions. The term "dsFv" is used herein to refer to an Fv with an engineered intermolecular disulfide bond to stabilize the V_{H}-V_{L} pair. A "F(ab')₂ " fragment is an antibody fragment essentially equivalent to that obtained from immunoglobulins (typically IgG) by digestion with an enzyme pepsin at pH 4.0-4.5. The fragment may be recombinantly produced. A "Fab"' fragment is an antibody fragment essentially equivalent to that obtained by reduction of the disulfide bridge or bridges joining the two heavy chain pieces in the F(ab')₂ fragment. The Fab' fragment may be recombinantly produced. A "Fab" fragment is an antibody fragment essentially equivalent to that obtained by digestion of immunoglobulins (typically IgG) with the enzyme papain. The Fab fragment may be recombinantly produced. The heavy chain segment of the Fab fragment is the Fd piece.

Prior to use of the device the analyte binding area may not be apparent to the user. In certain embodiments, the test result will be displayed as a plus sign or a minus sign, depending upon the presence or absence of analyte in the sample. When no analyte is present in the sample, the positive control area becomes visible as a minus sign, since the one or more components will change from a first color to a second color (e.g., from white to red). If analyte is present in the sample, the analyte reacts with the labeled reagent in the reagent zone and is captured in the analyte binding area by the specific binding molecules. In one embodiment the analyte binding area is two areas situated latitudinally on the assay strip on either side of the positive control area. By "latitudinal" is meant perpendicular to the direction of fluid flow through the device, which is usually also perpendicular to the length of the test strip. The positive control area and the analyte binding area interact with each other to produce a recognizable symbol. In one embodiment, the positive control area and analyte binding area interact to display a plus sign, but the analyte binding area can also be arranged with the positive control area to form other recognizable symbols.

In another embodiment, the analyte binding area is present as a single area present latitudinally across the test strip, and the positive control area is present as two areas situated on either side of the analyte binding area. The positive control area is thus present longitudinally on the test strip in two areas, and the analyte binding area is present either on top of the positive control area, or in between the positive control areas. In different embodiments these areas may or may not overlap. The one or more components used in the positive control area or filament can be selected to be of the same or similar colors, so that the positive control area and the analyte binding area will form a single symbol when they interact. Thus, the positive test symbol appears as a plus sign, and a negative test result produces a minus sign.

In yet another embodiment, a plus sign is formed by the positive control area and the analyte detection zone, which may or may not overlap. In this embodiment the analyte binding area is applied to the test strip before the positive control area is applied, providing a plus sign in the event of a positive result, and a minus sign in the event of a negative result.

In a further embodiment the positive control can be placed laterally on the test strip, and the analyte binding area placed longitudinally. In this orientation, the symbol for a positive result would still be a plus sign and a negative test result symbol would be a minus sign, simply oriented differently than in other embodiments.

In related embodiments, the positive control area is composed of multiple, aligned bars (instead of a single bar) that are perpendicular to and abut the analyte detection zone, and thus form a plus sign. Alternatively, the analyte binding area can be composed of multiple, aligned bars that are perpendicular to and abut the positive control area, which together form a plus sign.

In another embodiment, the test and positive control areas interact to form an "X." In this embodiment, the test and positive control areas are placed at an angle to the direction of sample flow. In a further embodiment, the test and positive control areas are arranged so that they interact to form a "Y."

### Type of Analytes

The analyte being assayed for presence or absence using the present invention can be any analyte. Examples of analytes that can be readily tested for using the present invention include (but are not limited to) human chorionic gonadotropin (hCG), luteinizing hormone (LH), follicle stimulating hormone (FSH), hepatitis C virus (HCV), hepatitis B virus, hepatitis B surface antigen, HIV, and any drug of abuse. Also, analyte can be detected in any liquid or liquefied sample such as, for example, urine, saliva, oral fluid, blood, plasma, or serum. Additional examples of analytes to be tested for include but are not limited to creatinine, bilirubin, nitrite, protein (nonspecific), blood, leukocytes, sugar, heavy metals or toxins, bacterial components (e.g. proteins or sugars specific to a particular type of bacteria, such as *E. coi*0157:H7*, S. aureus, Salmonella, C. perfringens, Campylobacter, L. monocytogenes, V. parahaemolyticus,* or *B.cereus*). Any other analyte that can be adapted to a lateral flow test format may also be incorporated into the present device.

### Types of Samples

Any sample type can be tested with the device of the present invention, including liquids of biological origin (e.g., urine and other body fluids, and clinical samples). Liquid samples may be derived from solid or semi-solid samples, including feces, biological tissue, and food samples. Such solid or semi-solid samples can be converted into a liquid sample by any suitable method, for example by mixing, chopping, macerating, incubating, dissolving or enzymatically digesting solid samples in a suitable liquid (e.g., water, phosphate-buffered saline, or other buffers). "Biological samples" include samples derived from living animals, plants, and food, including for example urine, saliva, blood and blood components, cerebrospinal fluid, vaginal swabs, semen, feces, sweat, exudates, tissue, organs, tumors, tissue and organ culture, cell cultures and conditioned media therefrom, whether from humans or animals. Food samples include samples from processed food components or final products, meat, cheese, wine, milk and drinking water. Plant samples include those derived from any plant, plant tissue, plant cell cultures and conditioned media therefrom. "Environmental samples" are those derived from the environment (e.g., a water sample from a lake or other body of water, effluent samples, soil samples, ground water, ocean water, and runoffwater. Sewage and related wastes can also be included as environmental samples.

### Methods of Use

The present invention also provides methods of using the devices of the invention to detect the presence or absence of an analyte in a liquid sample. The methods can include the steps of placing a liquid sample onto the sample application zone of a device of the present invention, and allowing the liquid sample to flow through the test strip. The liquid sample can be placed on the sample application zone by any convenient means, for example by using a dropper.

With reference to Figures 1-3, after application of liquid or liquefied sample to the sample application zone **25,** the sample begins to flow through the matrix and down the test strip. The sample enters a reagent zone **30** where reagents for conducting the assay and/or for labeling the analyte react with the sample. The analyte present in the sample is therefore labeled with a detectable label, in this case an antibody for the analyte carrying a gold sol particle. As the sample flows through the device, analyte contained in the fluid sample is labeled with a detectable label and is retained on the analyte binding area of the detection zone. The analyte binding area contains a member of a specific binding pair for a moiety associated with the analyte, in this case an antibody directed to an epitope directly on the analyte. In addition, the color-changing components contained in the positive control filament are wetted as liquid sample flows through the positive control area, and the filament changes from a first color (e.g., white) to a second color (e.g., red).

The positive control filament and the detectable label can be selected to have the same color, so that when labeled analyte binds to the analyte binding area, the interaction of the positive control area and the analyte control area results in the appearance of a recognizable symbol in the detection zone, in this case a "+" sign. In this case, as gold sol accumulates in the analyte binding area, it will produce a red appearance, which interacts with the red positive control filament to produce a "+" sign.

In cases where no analyte is present in the sample, the primary symbol (the minus sign of the positive control area) is apparent in the detection zone, resulting in a minus sign becoming visible after the assay is complete and indicating a negative result for the assay.

### Test Kits

Disclosed herein is a kit for determining the presence or absence of an analyte in a fluid, and instructions for use of the device. Test kits can be packaged in a variety of formats, depending upon the customer's needs.

In one embodiment, the test strips can be configured as "midstream" fertility test devices, having a housing enclosing the test strip, a wick in fluid communication with the sample application zone ofthe test strip and reagents for detecting a fertility hormone of interests, such as hCG, luteinizing hormone (LH) or follicle stimulating hormone (FSH). The housing has a window aligned with the detection zone, for viewing the test results. In certain embodiments, a kit containing midstream pregnancy devices has one or more individually wrapped devices and one set of instructions. The instructions explain how to perform the test and interpret the test results. For example, a patient provides a urine sample on a sample collection portion of a urine test device, which transmits the urine to a sample application pad on a device of the invention, The fluid passes through the reagent zone and detection zone of the device. If the test is negative (no pregnancy), a minus sign is uncovered when the color-changing component(s) in the positive control area have been wetted by sample, and therefore exhibit the second color. If the test is positive (pregnant), a plus sign is formed when the color changing component(s) in the positive control area interact with the color formed in the analyte binding area.

In various embodiments the kit contain 4 or more ovulation test devices or 6 or more ovulation test devices, one or more pregnancy test device(s), and an instruction booklet explaining use of the devices to identify the time of the LH surge, and how to use the devices to test for pregnancy. The devices can be any described herein. The devices can also be configured as a "cassette" for use in a professional laboratory.

In another embodiment, the kits contain test strips of the present invention. In one embodiment the test strips can be configured for pregnancy testing and packaged in containers having 15 or more test strips or 20 or more test strips, and an instruction insert. This type of kit is convenient for use in professional laboratories performing many pregnancy tests.

### Example 1 - Construction of hCG Test Device

This example describes the construction of one embodiment of the device of the invention for determining the presence or absence of hCG in a urine sample.

Test strips were constructed according to methods known in the art, except where otherwise noted. Referring to Figure 6, the positive control area was formed by placement of a positive control filament **50** within a groove **85** that was cut in a support layer **80** to receive the filament. The positive control filament **50** was an extension from a positive control pad **52.** In this embodiment the filament and control pad were provided in a double layer, as depicted in Figure 6. The filament was 0.9 mm x 10 mm and made of paper, and was previously treated with 3 µl of water-sensitive ink (purchased from Beijing Chuang-Zin Cheng-Ye Science and Technology Inc. Ltd., Beijing, China under the name "water-sensitive ink"), and dried at room temperature until it was white,

Goat anti-ahCG IgG (4.0 mg/ml) was applied at analyte binding areas on a nitrocellulose strip, which were located at either side of the positive control filament as the device is viewed from above, so that a plus sign would appear to the observer in the event of a positive assay result. The reagent zone contained gold sol labeled mouse anti-βhCG IgG, The portions of the test device were laminated together. Optionally, an anti-human IgG (1.3mg/ml) can be applied as a second procedural control line at the far end of the nitrocellulose using a microsyringe controlled by a microprocessor.

### Example 2 - Use of the Devices

After the test strips were constructed, they were tested using hCG positive and negative urine. Three lots of urine were obtained that had been correlated to a positive or negative group as containing an amount of hCG indicative of pregnancy. Lot 1 contained 200 urine samples. Lats 2 and 3 each contained 60 urine samples.

In each case 100% ofthe urine samples were correctly correlated to their group using the devices and methods of the present invention, for a specificity of greater than 99.9%.

The invention illustratively described herein may be practiced in the absence of any element or elements, limitation or limitations that are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by various embodiments and optional features, modification and variation of the concepts herein disclosed may be resorted to by those skilled in the art, and that such modifications and variations are considered to be within the scope of this invention as defined by the appended claims.

## Claims

1. A device for detecting the presence or absence of an analyte in a sample comprising:
a matrix that supports the flow of a liquid sample;
a sample application zone on the matrix for receiving a liquid sample;
a detection zone on the matrix having
a positive control area comprising a positive control filament containing one or more components that exhibit a first color when dry and a second color when wet, the one or more components comprising a colourless or pale-coloured basic dye selected from the group consisting of a triarylmethane-based dye, a diphenylmethane-based dye, a thazine-based dye, a lactam-based dye, and a fluoran-based dye, wherein the positive control filament is an independent structure on the device; and
an analyte binding area comprising a specific binding molecule; and
one or more reagent zones on the matrix comprising reagents for conducting the assay.

2. The device of claim 1, wherein the matrix is a nitrocellulose assay strip and the positive control area is comprised in the shape of a minus sign situated on the assay strip.

3. The device of any preceding claim, wherein the analyte binding area comprises two areas situated on either side of the positive control area having a specific binding molecule that binds to the analyte or to a molecule bound to the analyte, and aligned so that the positive control area and analyte binding area interact to form a recognizable symbol when the positive control area is wet and analyte is present in the sample.

4. The device of claim 3, wherein the recognizable symbol is a plus sign.

5. The device of any preceding claim, wherein the specific binding molecule is an antibody or antibody fragment.

6. The device of any preceding claim, wherein the analyte is selected from the group consisting of: human chorionic gonadotropin and luteinizing hormone.

7. The device of claim 6, wherein the analyte is human chorionic gonadotropin.

8. The device of any preceding claim, wherein the positive control area is demarcated by one or more positive control filaments present in the detection zone, and the positive control area does not comprise a member of a specific binding pair.

9. The device of any preceding claim, wherein the detection zone comprises an analyte binding area having a specific binding molecule for the analyte.

10. The device of claim 9, wherein the specific binding molecule comprises a label that provides a detectable signal.

11. The device of claim 10, wherein the label comprises a colored particle.

12. The device of claim 11, wherein the colored particle is a dextran bead,

13. The device of any one of claims 1, 2 and 5-12, wherein the analyte binding area comprises a bar situated latitudinally along the axis of the strip, and further comprises a specific binding molecule for the analyte or for a molecule bound to the analyte, and
the positive control area comprises two areas situated at either side of the analyte binding area, and the positive control area and analyte binding area interact to form a recognizable symbol.

14. The device of any preceding claim, wherein the first color is white and the second color is red.

15. The device of any one of claims 1-12 or claim 14, wherein the matrix is a test strip comprised of a bibulous material; the positive control area is comprised in the shape of a minus sign situated longitudinally along the axis of the bibulous material; and the analyte binding area comprises two areas situated on either side of the positive control area, and the analyte binding area and positive control area interact to form a recognizable symbol.

16. The device of claim 15, wherein the analyte binding area comprises a bar situated latitudinally along the axis of the strip, and wherein the analyte binding area comprises a specific binding molecule for the analyte bound to a label.

17. A method of determining the presence or absence of an analyte in a liquid sample comprising; placing the liquid sample onto the device of any preceding claim;
allowing the liquid sample to flow through the matrix and thereby pass through the one or more reagent zones so that reagents for conducting the assay react with the liquid sample to form a detectable reaction product when analyte is present in the liquid sample;
allowing the liquid sample to flow through the detection zone thereby wetting the positive control filament comprising one or more components that exhibit a first color when dry and a second color when wet, wherein analyte contained in the sample is restrained on the analyte binding area as sample flows through the detection zone; and
observing the detection zone of the device to determine the presence or absence of analyte in the liquid sample.

18. The method of claim 17, wherein analyte present in the sample is labeled with a detectable label as it passes through the reagent zone to form a labeled analyte, and labeled analyte is retained at the analyte binding area as sample passes through the detection zone.

## Patentansprüche

1. Vorrichtung zum Ermitteln des Vorhandenseins oder des Nichtvorhandenseins eines Analyten in einer Probe, die aufweist:
eine Matrix, die das Fließen einer flüssigen Probe unterstützt;
eine Probenaufbringzone auf der Matrix zur Aufnahme einer flüssigen Probe;
eine Ermittlungszone auf der Matrix mit
einem positiven Kontrollbereich, der ein positives Kontrollfilament aufweist, das einen oder mehrere Komponenten enthält, die eine erste Farbe haben, wenn sie trocken sind, und eine zweite Farbe, wenn sie nass sind, wobei die eine oder die mehreren Komponenten einen farblosen oder blassfarbenen Grundfarbstoff haben, der ausgewählt wird aus der Gruppe bestehend aus einem triarylmethanbasierten Farbstoff, einem diphenylmethanbasierten Farbstoff, einem thiazinbasierten Farbstoff, einem lactambasierten Farbstoff und einem fluoranbasierten Farbstoff, wobei das positive Kontrollfilament eine unabhängige Struktur auf der Vorrichtung ist; und
einem Analytbindungsbereich, der ein spezifisches Bindungsmolekül aufweist; und
eine oder mehrere Reagenzzonen auf der Matrix, die Reagenzien zum Durchführen der Untersuchung aufweisen.

2. Vorrichtung nach Anspruch 1, wobei die Matrix ein Nitrocellulose-Probestreifen ist und der positive Kontrollbereich in Form eines Minuszeichens vorliegt, das sich auf dem Probestreifen befindet.

3. Vorrichtung nach einem der vorhergehenden Ansprüche, wobei der Analytbindungsbereich zwei Bereiche aufweist, die sich auf jeder Seite des positiven Kontrollbereichs befinden, mit einem spezifischen Bindungsmolekül, das sich an den Analyten oder an ein Molekül bindet, das an den Analyten gebunden ist, und die derart ausgerichtet sind, dass der positive Kontrollbereich und der Analytbindungsbereich wechselwirken, um ein erkennbares Symbol zu bilden, wenn der positive Kontrollbereich nass ist und ein Analyt in der Probe vorhanden ist.

4. Vorrichtung nach Anspruch 3, wobei das erkennbare Symbol ein Pluszeichen ist.

5. Vorrichtung nach einem der vorhergehenden Ansprüche, wobei das spezifische Bindungsmolekül ein Antikörper oder ein Antikörperfragment ist.

6. Vorrichtung nach einem der vorhergehenden Ansprüche, wobei der Analyt ausgewählt wird aus der Gruppe bestehend aus: humanem Choriongonadotropin und luteinisierendem Hormon.

7. Vorrichtung nach einem der vorhergehenden Ansprüche, wobei der Analyt humanes Choriongonadotropin ist.

8. Vorrichtung nach einem der vorhergehenden Ansprüche, wobei der positive Kontrollbereich von einem oder mehreren positiven Kontrollfilamenten abgegrenzt wird, die in der Ermittlungszone vorhanden sind, und der positive Kontrollbereich kein Teil eines spezifischen Bindungspaars aufweist.

9. Vorrichtung nach einem der vorhergehenden Ansprüche, wobei die Ermittlungszone einen Analytbindungsbereich aufweist, der ein spezifisches Bindungsmolekül für den Analyten hat.

10. Vorrichtung nach Anspruch 9, wobei das spezifische Bindungsmolekül ein Kennzeichen aufweist, das ein ermittelbares Signal bereitstellt.

11. Vorrichtung nach Anspruch 10, wobei das Kennzeichen ein farbiges Partikel aufweist.

12. Vorrichtung nach Anspruch 11, wobei das farbige Partikel ein Dextrantropfen ist.

13. Vorrichtung nach einem der Ansprüche 1, 2 und 5-12, wobei der Analytbindungsbereich einen Balken aufweist, der sich der Breite nach entlang der Achse des Streifens erstreckt, und des Weiteren ein spezifisches Bindungsmolekül für den Analyten oder für ein Molekül, das an den Analyten gebunden ist, aufweist, und
der positive Kontrollbereich zwei Bereiche aufweist, die sich auf jeder Seite des Analytbindungsbereichs befinden, und der positive Kontrollbereich und der Analytbindungsbereich wechselwirken, um ein erkennbares Symbol zu bilden.

14. Vorrichtung nach einem der vorhergehenden Ansprüche, wobei die erste Farbe weiß und die zweite Farbe rot ist.

15. Vorrichtung nach einem der Ansprüche 1-12 oder 14, wobei die Matrix ein Teststreifen ist, der aus einem saugfähigen Material besteht; der positive Kontrollbereich in Form eines Minuszeichens vorliegt, das sich der Länge nach entlang der Achse des saugfähigen Materials befindet; und der Analytbindungsbereich zwei Bereiche aufweist, die sich auf jeder Seite des positiven Kontrollbereichs befinden, und der Analytbindungsbereich und der positive Kontrollbereich wechselwirken, um ein erkennbares Symbol zu bilden.

16. Vorrichtung nach Anspruch 15, wobei der Analytbindungsbereich einen Balken aufweist, der sich der Breite nach entlang der Achse des Streifens befindet, und wobei der Analytbindungsbereich ein spezifisches Bindungsmolekül für den Analyten aufweist, das an ein Kennzeichen gebunden ist.

17. Verfahren zum Ermitteln des Vorhandenseins oder des Nichtvorhandenseins eines Analyten in einer flüssigen Probe, das umfasst:
Platzieren der flüssigen Probe auf der Vorrichtung nach einem der vorhergehenden Ansprüche;
Gestatten, dass die flüssige Probe durch die Matrix fließt und dabei durch die eine oder mehrere Reagenzzonen hindurchgeht, so dass Reagenzien zum Durchführen der Untersuchung mit der flüssigen Probe reagieren, um ein ermittelbares Reaktionsprodukt zu bilden, wenn ein Analyt in der flüssigen Probe vorhanden ist;
Gestatten, dass die flüssige Probe durch die Ermittlungszone fließt und dabei das positive Kontrollfilament benetzt, das eine oder mehrere Komponenten aufweist, die eine erste Farbe haben, wenn sie trocken sind, und eine zweite Farbe, wenn sie nass sind, wobei ein Analyt, der in der Probe enthalten ist, auf dem Analytbindungsbereich zurückgehalten wird, während die Probe durch die Ermittlungszone fließt; und
Überwachen der Ermittlungszone der Vorrichtung, um das Vorhandensein oder das Nichtvorhandensein eines Analyten in der flüssigen Probe zu bestimmen.

18. Verfahren nach Anspruch 17, wobei ein Analyt, der in der Probe vorhanden ist, mit einem erkennbaren Kennzeichen gekennzeichnet wird, während er durch die Reagenzzone hindurchgeht, um einen gekennzeichneten Analyten zu bilden, und der gekennzeichnete Analyt an dem Analytbindungsbereich zurückgehalten wird, während die Probe durch die Ermittlungszone hindurchgeht.

## Revendications

1. Dispositif pour détecter la présence ou l'absence d'un analyte dans un échantillon comprenant :
- une matrice qui supporte l'écoulement d'un échantillon liquide ;
- une zone d'application d'échantillon sur la matrice pour recevoir un échantillon liquide ;
- une zone de détection sur la matrice ayant :
- une région de contrôle positif comprenant un filament de contrôle positif contenant un ou plusieurs composants qui présentent une première couleur lorsqu'ils sont secs et une seconde couleur lorsqu'ils sont humides, le ou les composants comprenant un colorant basique incolore ou à coloration pâle choisi dans le groupe consistant en un colorant à base de triarylméthane, un colorant à base de diphénylméthane, un colorant à base de thazine, un colorant à base de lactame et un colorant à base de fluorane, le filament de contrôle positif étant une structure indépendante sur le dispositif ; et
- une région de liaison de l'analyte comprenant une molécule de liaison spécifique ; et
- une ou plusieurs zones de réactifs sur la matrice comprenant des réactifs pour effectuer l'essai.

2. Dispositif selon la revendication 1, dans lequel la matrice est une bande d'essai de nitrocellulose et la région de contrôle positif est comprise dans la forme d'un signe moins situé sur la bande d'essai.

3. Dispositif selon l'une quelconque des revendications précédentes, dans lequel la région de liaison de l'analyte comprend deux régions situées sur l'un et l'autre côté de la région de contrôle positif ayant une molécule de liaison spécifique qui se lie à l'analyte ou à une molécule liée à l'analyte, et alignées de telle sorte que la région de contrôle positif et la région de liaison de l'analyte interagissent pour former un symbole reconnaissable lorsque la région de contrôle positif est humide et que l'analyte est présent dans l'échantillon.

4. Dispositif selon la revendication 3, dans lequel le symbole reconnaissable est un signe plus.

5. Dispositif selon l'une quelconque des revendications précédentes, dans lequel la molécule de liaison spécifique est un anticorps ou un fragment d'anticorps.

6. Dispositif selon l'une quelconque des revendications précédentes, dans lequel l'analyte est choisi dans le groupe consistant en : gonadotrophine chorionique humaine et hormone lutéinisante.

7. Dispositif selon la revendication 6, dans lequel l'analyte est la gonadotrophine chorionique humaine.

8. Dispositif selon l'une quelconque des revendications précédentes, dans lequel la région de contrôle positif est délimitée par un ou plusieurs filaments de contrôle positif présents dans la zone de détection, et la région de contrôle positif ne comprend pas d'élément d'une paire de liaison spécifique.

9. Dispositif selon l'une quelconque des revendications précédentes, dans lequel la zone de détection comprend une région de liaison de l'analyte ayant une molécule de liaison spécifique pour l'analyte.

10. Dispositif selon la revendication 9, dans lequel la molécule de liaison spécifique comprend un marqueur qui fournit un signal détectable.

11. Dispositif selon la revendication 10, dans lequel le marqueur comprend une particule colorée.

12. Dispositif selon la revendication 11, dans lequel la particule colorée est une bille de dextrane.

13. Dispositif selon l'une quelconque des revendications 1, 2 et 5-12, dans lequel la région de liaison de l'analyte comprend une barre située latitudinalement le long de l'axe de la bande, et comprend de plus une molécule de liaison spécifique pour l'analyte ou pour une molécule liée à l'analyte, et
la région de contrôle positif comprend deux régions situées de l'un et l'autre côté de la région de liaison de l'analyte, et la région de contrôle positif et la région de liaison de l'analyte interagissent pour former un symbole reconnaissable.

14. Dispositif selon l'une quelconque des revendications précédentes, dans lequel la première couleur est le blanc et la seconde couleur est le rouge.

15. Dispositif selon l'une quelconque des revendications 1-12 ou la revendication 14, dans lequel la matrice est une bande de test constituée d'une matière siccative ; la région de contrôle positif est comprise dans la forme d'un signe moins situé de façon longitudinale le long de l'axe de la matière siccative ; et la région de liaison de l'analyte comprend deux régions situées de l'un et l'autre côté de la région de contrôle positif, et la région de liaison de l'analyte et la région de contrôle positif interagissent pour former un symbole reconnaissable.

16. Dispositif selon la revendication 15, dans lequel la région de liaison de l'analyte comprend une barre située latitudinalement le long de l'axe de la bande, et dans lequel la région de liaison de l'analyte comprend une molécule de liaison spécifique pour l'analyte lié à un marqueur.

17. Procédé de détermination de la présence ou de l'absence d'un analyte dans un échantillon liquide comprenant :
- placer l'échantillon liquide sur le dispositif selon l'une quelconque des revendications précédentes ;
- amener l'échantillon liquide à s'écouler à travers la matrice et par là à passer par la ou les zones de réactif de telle sorte les réactifs pour effectuer l'essai réagissent avec l'échantillon liquide pour former un produit de réaction détectable lorsque l'analyte est présent dans l'échantillon liquide ;
- amener l'échantillon liquide à s'écouler à travers la zone de détection mouillant de ce fait le filament de contrôle positif comprenant un ou plusieurs composants qui présentent une première couleur lorsqu'ils sont secs et une seconde couleur lorsqu'ils sont humides, l'analyte contenu dans l'échantillon étant retenu sur la région de liaison de l'analyte à mesure que l'échantillon s'écoule à travers la zone de détection ; et
- observer la zone de détection du dispositif pour déterminer la présence ou l'absence de l'analyte dans l'échantillon liquide.

18. Procédé selon la revendication 17, dans lequel l'analyte présent dans l'échantillon est marqué par un marqueur détectable à mesure qu'il passe à travers la zone de réactif pour former un analyte marqué, et l'analyte marqué est retenu à la région de liaison d'analyte à mesure que l'échantillon passe à travers la zone de détection.
